# EUROPÄISCHE PATENTANMELDUNG

(11) **EP 4 775 662 A1**
(43) Veröffentlichungstag der Anmeldung: **15.07.2026**
(21) Anmeldenummer: 25151611.8
(22) Anmeldetag: 13.01.2025
(51) Int. Cl.: C12N 1/14, A23J 1/00, C12R 1/69

(54) **VERFAHREN ZUR FERMENTATIVEN HERSTELLUNG VON PILZMASSE FÜR LEBENSMITTEL**

(71) Anmelder: Kynda Biotech GmbH, 29585 Jelmstorf (DE)
(72) Erfinder: SCHNABEL, Franziskus, 29585 Jelmstorf (DE)
(74) Vertreter: Taruttis, Stefan Georg

(57) **Zusammenfassung**

Verfahren zur fermentativen Herstellung von Pilzmycel durch Bereitstellen eines wässrigen Nährmediums mit einem Gehalt an Substrat, das pflanzliches Protein enthält, Inokulieren des Nährmediums mit zumindest einem Pilz, Durchmischen und Belüften des inokulierten Nährmediums zum Anziehen von Pilzmasse, wobei dem Nährmedium Ammoniumionen zugesetzt werden, so dass der pH-Wert ohne kontrolliertes Einstellen des pH-Werts durch die Fermentation um zumindest 2 pH-Einheiten erniedrigt wird, Abtrennen von Pilzmasse aus der Fermenterbrühe zur Herstellung wässriger Pilzmasse und eines wässrigen Rückstands, Bereitstellen eines weiteren wässrigen Nährmediums durch Mischen zumindest eines Teils des wässrigen Rückstands, und Wiederholung der vorgenannten Schritte.

## Beschreibung

Die vorliegende Erfindung betrifft ein Verfahren zur fermentativen Herstellung von Pilzmasse, die als Lebensmittel geeignet ist. Die bei der Herstellung durchgeführte Fermentation ist bevorzugt eine Submersfermentation in einem Bioreaktor bei Belüftung, bevorzugt unter Rühren der Fermentationsbrühe.

Das Verfahren hat den Vorteil, dass das zu fermentierende Substrat nicht notwendigerweise steril ist, insbesondere vor der Fermentation nicht notwendigerweise autoklaviert wird, sondern bevorzugt nur keimreduziert ist, z.B. vor der Fermentation teilentkeimt wird, z.B. pasteurisiert wird. Vorteilhaft ergibt sich daraus ein Energiebedarf, der für die Pasteurisation geringer ist als für die Sterilisation durch Autoklavieren. Das Substrat weist bevorzugt pflanzliches Protein und/oder pflanzliche Kohlenhydrate, optional Pflanzenfasern, z.B. bevorzugt pflanzliche Nebenprodukte der Lebensmittelherstellung auf oder besteht daraus. Die WO 2020/061502 A1 beschreibt ein aerobes Fermentationsverfahren mit Pilzmycel zur Herstellung von Fleischanaloga. Dabei wird ein filamentöser Pilz in Nährmedium kultiviert, geerntet, optional pasteurisiert, extrudiert und getrocknet. Das Nährmedium kann ein Nebenprodukt der Lebensmittelproduktion sein.

Die US 2009/0148558 A1 beschreibt ein Verfahren zur Herstellung von Fleischanaloga aus Pilzmycel, das in einer synthetischen Nährbrühe aus Kartoffel-Dextrose-Brühe, Hefeextrakt, Malzextrakt und Soytone (mit Papain verdautes Sojamehl) mit Zuckerrohrextrakt und Natriumnitrat angezogen wird.

Der Erfindung stellt sich die Aufgabe, ein Verfahren zur Herstellung von Pilzmasse bereitzustellen, das einen geringen Energieaufwand benötigt, insbesondere nicht die Sterilisation des Substrats beispielsweise durch Autoklavieren erfordert. Bevorzugt soll das Verfahren einen Schritt der Submersfermentation aufweisen, die ohne Steuerung des pH-Werts durchgeführt wird.

Die Erfindung löst die Aufgabe mit den Merkmalen der Ansprüche und insbesondere mittels eines Verfahrens zur Herstellung von Pilzmasse durch Fermentation, bevorzugt Submersfermentation, das die Schritte
1) Bereitstellen eines wässrigen Nährmediums mit einem Gehalt an Substrat, das pflanzliches Protein und/oder Kohlenhydrat enthält, wobei das Substrat optional pasteurisiert ist,
2) Inokulieren des Nährmediums mit zumindest einem Pilz zur Erzeugung eines mit dem zumindest einen Pilz inokulierten Nährmediums,
3) Durchmischen und Belüften des inokulierten Nährmediums zum Anziehen von Pilzmasse des zumindest einen Pilzes zur aeroben fermentativen Herstellung von Fermenterbrühe, die angezogene Pilzmasse enthält, wobei dem Nährmedium Ammoniumionen zugeben werden, wobei der pH-Wert durch die Fermentation um zumindest 2 pH-Einheiten, bevorzugter um zumindest 3 pH-Einheiten oder um zumindest 4 pH-Einheiten, bevorzugter um zumindest 4,5 oder zumindest 5 pH-Einheiten erniedrigt wird,
4) bevorzugt ohne kontrolliertes Einstellen des pH-Werts, z.B. ohne Einstellen des pH-Werts mittels des kontrollierten Zusetzens von Säure,
5) Abtrennen von Pilzmasse aus der Fermenterbrühe zur Herstellung wässriger Pilzmasse und zumindest eines wässrigen Rückstands,
6) bevorzugt Bereitstellen eines weiteren wässrigen Nährmediums durch Mischen zumindest eines Teils des wässrigen Rückstands, der optional unlösliche pflanzliche Bestandteile enthält oder aus löslichen Bestandteilen besteht, mit pflanzlichem Protein und/oder Kohlenhydrat und Wiederholung der voranstehenden Schritte, insbesondere Wiederholung der Schritte 2) bis 6),
aufweist oder daraus besteht.

Das Verfahren zur fermentativen Herstellung von Pilzmycel umfasst generell das Bereitstellen eines wässrigen Nährmediums mit einem Gehalt an Substrat, das pflanzliches Protein enthält, Inokulieren des Nährmediums mit zumindest einem Pilz, Durchmischen und Belüften des inokulierten Nährmediums zum Anziehen von Pilzmasse mittels aerober Fermentation, wobei dem Nährmedium Ammoniumionen zugesetzt werden, so dass der pH-Wert ohne kontrolliertes Einstellen des pH-Werts durch die aerobe Fermentation um zumindest 2 pH-Einheiten erniedrigt wird, Abtrennen von Pilzmasse aus der Fermenterbrühe zur Herstellung wässriger Pilzmasse und eines wässrigen Rückstands, Bereitstellen eines weiteren wässrigen Nährmediums und Einmischen zumindest eines Teils des wässrigen Rückstands, und bevorzugt Wiederholung der vorgenannten Schritte, oder besteht daraus.

Das Verfahren hat den Vorteil, dass der Pilz ein zur Herstellung von Lebensmitteln geeigneter ist, z.B. ein durch die zuständigen Behörden für die Herstellung von Lebensmitteln zugelassener Pilz ist, auch als GRAS bezeichnet. Insbesondere ist der im Verfahren eingesetzte Pilz nicht genetisch manipuliert oder mutiert.

Das Substrat ist optional pasteurisiert, bevorzugt nicht sterilisiert, sondern kann ausschließlich pasteurisiert sein. Bevorzugt hat das Substrat vor dem Inokulieren von Schritt 2) einen Keimgehalt, der der Keimgehalt aus der Herstellung ist, z.B. der Keimgehalt von Okara, das bevorzugt frisch ist, optional eingefroren oder nicht gefroren für bis zu 7 Tage oder bis zu 5 oder bis 3 Tage bei maximal 8°C, bevorzugt maximal 5 °C oder maximal 3°C gelagert ist. Das Substrat kann pflanzliches Protein und/oder Kohlenhydrat aufweisen, z.B. in Mischung mit Cellulose, Hemicellulose und/oder Fett, oder aus pflanzlichem Protein und/oder Kohlenhydrat bestehen.

Das pflanzliche Protein und/oder Kohlenhydrat kann der wasserunlösliche Rückstand aus der wässrigen Extraktion von Hülsenfrüchten oder Getreide sein, der auch Okara genannt wird, z.B. Rückstand aus der wässrigen Extraktion von Bohnen, insbesondere Sojabohnen, Weizen, Reis oder Linsen, insbesondere wasserunlöslicher Rückstand aus der Herstellung von Tofu aus Sojabohnen, der auch Sojaokara genannt wird.

Alternativ oder zusätzlich kann das Substrat Getreide- und Leguminosenstroh, Zuckerrübenschnitzel, insbesondere nach der Extraktion von Zucker, Getreidekleie, z.B. Weizenkleie, Maiskleie und Reiskleie, Kartoffelschalen, Apfelschalen, Traubenschalen, Citrusschalen und Karottenschalen, Blumenkohlschnitt, Lauchgrün, ausextrahierte Teeblätter, Presskuchen aus der Speiseölgewinnung, insbesondere Rapspresskuchen, Sojapresskuchen und Olivenpresskuchen, leere Sonnenblumenblüten, Sonnenblumenschalen, Nussschalen, optional gemahlen oder ungemahlen und stückig, Maiskolben nach dem Entfernen der Maiskörner, rote Erdnussschalen, Biertreber, Weintrester, optional nach dem Abdestillieren von Alkohol, wie z.B. nach Herstellung von Grappa zurückgebliebener Weintrester, Zuckerrohrbagasse, Zuckerrohrblätter, Kaffeesatz, z.B. nach einer wässrigen Extraktion und/oder nach dem Extrahieren von Koffein, Rück- und Restbrot, Bananenblätter oder eine Kombination von zumindest zweien dieser aufweisen oder daraus bestehen.

Zusätzlich oder als Alternative dazu, dass das wässrige Nährmedium einen Gehalt an pasteurisiertem oder keimreduziertem Substrat enthält, kann die wässrige Phase des wässrigen Nährmediums separat vor dem Einbringen des Substrats oder nach Einbringen des Substrats pasteurisiert oder teilentkeimt werden. Bevorzugt wird dem Nährmedium zumindest ein Teil des wässrigen Rückstands, optional vor oder nach dem optionalen Schritt des Pasteurisierens oder der Keimreduktion, zugesetzt, wobei der wässrige Rückstand derjenige ist, der aus einer mit dem Verfahren hergestellten Fermenterbrühe abgetrennt wurde, so dass dieser wässrige Rückstand das Nährmedium und darin enthaltenes Substrat ansäuert.

Es hat sich gezeigt, dass die fermentative Herstellung von Fermenterbrühe durch Durchmischen und Belüften des Nährmediums, das mit einem Pilz inokuliert wurde, bei Zusetzen von Ammoniumionen als anorganischer Stickstoffquelle, bevorzugt als einzige anorganische Stickstoffquelle, z.B. als Ammoniumsulfat, zur Erniedrigung des pH-Werts der Fermenterbrühe um zumindest 1 pH-Einheit, bevorzugt um zumindest 2 pH-Einheiten, bevorzugter um zumindest 3 pH-Einheiten oder um zumindest 4 pH-Einheiten, bevorzugter um zumindest 4,5 bis 5 pH-Einheiten führt, ohne dass der pH-Wert mittels Zugabe von Säure gesteuert wird. Dabei enthält das Substrat bevorzugt den wasserunlöslichen Rückstand aus der wässrigen Extraktion von Hülsenfrüchten oder Getreide oder besteht daraus. Entsprechend wird erfindungsgemäß der pH-Wert der Fermenterbrühe, der Ammoniumionen zugesetzt sind, während der Fermentation um zumindest 1 pH-Einheit, bevorzugt um zumindest 2 pH-Einheiten, bevorzugter um zumindest 3 pH-Einheiten oder um zumindest 4 pH-Einheiten, bevorzugter um zumindest 4,5 oder zumindest 5 pH-Einheiten erniedrigt, optional zusätzlich durch Zusetzen von Säure, wobei bevorzugt der pH-Wert allein durch den zugesetzten Gehalt an Ammoniumionen während der Fermentation erniedrigt wird, insbesondere innerhalb von 48 h, bevorzugt innerhalb von 36 h oder innerhalb von 24 h nach Beginn der Fermentation, z.B. gestartet durch Inokulieren des Nährmediums. Bevorzugt wird der pH-Wert der Fermenterbrühe durch das Anziehen des Pilzes auf einen pH-Wert von 2,5 bis 5, z.B. 3,5 bis 4,5 erniedrigt, insbesondere bevorzugt innerhalb von 48 h, bevorzugt innerhalb von 36 h oder innerhalb von 24 h nach Beginn der Fermentation. Ammoniumionen, als Salz oder in Lösung, werden z.B. zum Erreichen einer Konzentration im Nährmedium von 0,03 bis 0,1 M, bevorzugt 0,04 bis 0,9 M, bevorzugter 0,05 bis 0,085 M, z.B. 0,06 bis 0,08 oder bis 0,076 M (0,076 M entspricht 10 g/L Ammoniumsulfat) zugesetzt.

Daher wird bei der fermentativen Herstellung erfindungsgemäß der pH-Wert der Fermenterbrühe durch den Pilz während Fermentation mittels Zugabe von Ammoniumionen um zumindest 1 pH-Einheit, bevorzugt um zumindest 2 pH-Einheiten, bevorzugter um zumindest 3 pH-Einheiten oder um zumindest 4 pH-Einheiten oder um zumindest 5 pH-Einheiten erniedrigt. Bevorzugt besteht die anorganische Stickstoffquelle, die dem Nährmedium zugesetzt wird, aus Ammoniumionen, insbesondere bei einem Pilz, der aus der Gruppe von Aspergillus oryzae (z.B. *White Koji, Weißer Koji*) oder Aspergillus sojae (z.B. Yellow Koji, Gelber Koji) ausgewählt ist. Denn es hat sich gezeigt, dass z.B. bei der Fermentation durch Aspergillus oryzae (*White Koji*), wenn dem Substrat, das im Wesentlichen aus 30 g/L Okara aus Soja in Wasser besteht, Harnstoff oder Natriumnitrat als einzige anorganische Stickstoffquelle zugesetzt ist, oder keine anorganische Stickstoffquelle zugesetzt wird, der pH-Wert von anfänglich 7 bis 9 nur geringfügig sinkt, gleichbleibt oder steigt.

Das Pasteurisieren des Substrats erfolgt soweit, dass Bakterien und Hefen sowie Schimmelpilze, die im Substrat enthalten sind, auf maximal 10⁶ KBE/mL, bevorzugt maximal 10⁵ KBE/mL oder maximal 10⁴ KBE/mL verringert sind, noch bevorzugter inaktiviert sind. Das Pasteurisieren bzw. Teilentkeimen kann durch Wärmebehandlung, z.B. mittels Dampfinjektion, durch Behandeln mit gepulsten elektrischen Feldern, oder eine Kombination dieser erfolgen. Das Substrat kann vor oder nach dem Pasteurisieren in Wasser suspendiert werden, um das Nährmedium bereitzustellen.

Bevorzugt wird während oder nach der Fermentation Pilzmasse aus der Fermenterbrühe abgetrennt, um einen wässrigen Rückstand zu erhalten, der optional unlösliche Bestandteile enthält oder aus löslichen Bestandteilen besteht. Das Abtrennen von Pilzmasse aus der Fermenterbrühe kann durch Abtrennen eines Teils der wässrigen Phase aus der Fermenterbrühe, z.B. einer wässrigen Phase, die im Wesentlichen keine unlöslichen Bestandteile enthält, erfolgen. Die wässrige Phase, die aus der Fermenterbrühe abgetrennt ist oder nach Abtrennen von Pilzmasse zurückbleibt weist bevorzugt einen pH-Wert von maximal 5,5, maximal 5,0, maximal 4,5, bevorzugter von maximal 4,0, maximal 3,5, oder maximal 3,0, noch bevorzugter von maximal 2,5 auf.

In einer bevorzugten Ausführungsform wird diese wässrige Phase mit pflanzlichem Protein und/oder Kohlenhydrat gemischt, um Nährmedium bereitzustellen, bevorzugt mit Zusetzen von Ammoniumionen, optional ohne Pasteurisieren oder Teilentkeimen, auch als Keimreduzieren bezeichnet. Optional kann das pflanzliche Protein und/oder Kohlenhydrat anschließend an das Mischen mit dieser wässrigen Phase pasteurisiert werden. Bevorzugt wird das pflanzliche Protein und/oder Kohlenhydrat vor dem Mischen mit dieser wässrigen Phase pasteurisiert, da sich gezeigt hat, dass das Mischen dieser wässrigen Phase, die aus einer Fermentation stammt, zu einer schnelleren anschließenden fermentativen Herstellung von Fermenterbrühe führt, die angezogene Pilzmasse enthält. Gegenwärtig wird angenommen, dass die schnellere fermentative Herstellung von Fermenterbrühe, die angezogene Pilzmasse enthält, wenn das Nährmedium eine aus einer Fermenterbrühe abgetrennte wässrige Phase in Mischung mit pflanzlichem Protein und/oder Kohlenhydrat enthält, auf die Erniedrigung des pH-Werts und auf den Gehalt der wässrigen Phase an hydrolysierenden Enzymen zurückgeht.

Das Animpfen des Substrats mit einem Pilz kann vor oder nach dem Suspendieren des pasteurisierten Substrats in Wasser, das bevorzugt zumindest zum Teil aus wässriger Phase besteht, die aus einer Fermenterbrühe abgetrennt wurde, erfolgen, z.B. durch Einmischen von Sporen oder Mycel eines Pilzes. Bevorzugt erfolgt das Animpfen mit Mycel im Verhältnis 1:5 bis 1:25, z.B. 1:10 bis 1:20 Vol./Vol. zum Substrat oder zum suspendierten Substrat. Das zum Animpfen eingesetzte Mycel ist bevorzugt durch aerobe Submersfermentation hergestellt, z.B. in wässrigem Nährmedium, das synthetisch ist und/oder eine wässrige Suspension eines erfindungsgemäß eingesetzten Substrats ist.

Es hat sich gezeigt, dass es ausreicht, wenn das Substrat, optional das Nährmedium einschließlich des Substrats, vor dem Animpfen pasteurisiert wird, z.B. durch Pasteurisieren bei 68 °C für 20 bis 60 min, da der zumindest eine Pilz nach dem Animpfen nicht von Bakterien oder anderen Pilzen zurückgedrängt wird, z.B. nicht überwachsen, bevorzugt nicht von Bakterien oder anderen Pilzen kontaminiert wird. Bevorzugt wird das zum Animpfen eingesetzte Mycel vor dem Animpfen zerkleinert, z.B. durch intensives Scheren wie z.B. mittels eines Ultra-Turrax bei 6400 bis 13000 Upm für 3 bis 30 s, insbesondere intensives Scheren unmittelbar vor dem Animpfen, z.B. innerhalb maximal 10 min oder maximal 5 min vor dem Animpfen. Das intensive Scheren erfolgt, bis das zum Animpfen vorgesehene Mycel zu einzelnen Hyphen oder Aggregaten von maximal 10 Hyphen, bevorzugter maximal 5 oder maximal 3 Hyphen zerkleinert ist, wie z.B. mittels mikroskopischer Kontrolle feststellbar ist.

Bevorzugt hat das Substrat einen Anteil von 3 bis 40 Gew.-%, bevorzugt 20 bis 40 Gew.-% oder 20 bis 30 Gew.-% Trockensubstanz in der wässrigen Suspension. Es hat sich gezeigt, dass bei einem solchen Anteil des Substrats in wässriger Suspension bei oder vor dem Animpfen durch das Fermentieren als Fermentationsbrühe im Wesentlichen Mycel gebildet wird, das noch pumpfähig ist, wobei die Fermentationsbrühe jedoch kaum einen oder keinen freien wässrigen Anteil aufweist. Das erzeugte Mycel hat dabei z.B. eine Trockensubstanz von ca. 10 bis 30 g/L.

Das Fermentieren im Submersverfahren erfolgt z.B. in einem geschlossenen Behälter, hier auch als Reaktor bezeichnet, der mit keimarmer oder steriler Luft belüftet wird, während das in dem Nährmedium suspendierte Substrat mit einem Rührer oder allein durch die Belüftung durchmischt wird. Die Belüftung erfolgt bevorzugt, bis die Gelöstsauerstoffkonzentration zumindest 10 %, bevorzugt zumindest 20 % beträgt. Der Rührer kann ein Turbinen-, Propeller- oder Ankerrührer sein. Bevorzugt weist der Behälter eine Temperiereinrichtung auf, insbesondere eine Kühleinrichtung. Der Behälter, in dem die Fermentation abläuft, kann z.B. gekühlt werden, um die Fermenterbrühe während der Fermentation auf eine konstante Temperatur von 15°C bis 30°C zu kühlen.

Bevorzugt bewirkt nur die Belüftung das Mischen und der Behälter weist keinen mechanisch oder magnetisch angetriebenen Rührer auf. Es hat sich gezeigt, dass der Pilz, insbesondere wenn er vor dem Animpfen durch Scherung zerkleinert wurde, auch bei Belüftung mit unsteriler Luft nicht von anderen Mikroorganismen überwachsen oder beeinträchtigt wird und bevorzugt der Pilz andere Mikroorganismen unterdrückt. Weiter bevorzugt kann das Fermentieren im offenen Behälter erfolgen. Optional wird der Behälter allein an seiner Außenwand durch Umgebungsluft temperiert, insbesondere gekühlt.

Die Fermentation wird bevorzugt durchgeführt, bis die wässrige Fraktion der Fermenterbrühe keine unlöslichen Reste des Substrats enthält, wobei unlösliche Reste z.B. solche sind, die durch ein Sieb mit einer Maschengröße von 1 mm, bevorzugt maximal 0,5 mm oder maximal 0,2 mm oder maximal 0,05 mm zurückgehalten werden.

Insbesondere wird die Fermentation durchgeführt, bis der Pilz das Substrat zumindest soweit abgebaut hat, dass deren Reste eine maximale Größe von 1 mm, bevorzugt maximal 0,5 mm oder maximal 0,2 mm aufweisen. Bevorzugt wird die Fermentation fortgesetzt, bis das Substrat vollständig abgebaut ist, bzw. es wird erst dann die Fermenterbrühe abgezogen, wenn das Substrat vollständig abgebaut ist.

Generell kann der Anteil der wässrigen Fraktion der Fermenterbrühe, der nach Abtrennen von Mycel verbleibt, durch Sieben von Resten befreit werden, die größer sind, als maximal 1 mm, bevorzugt maximal 0,5 mm oder maximal 0,2 mm.

Generell ist bevorzugt, die Fermentation als Batch-Verfahren oder Fed-batch-Verfahren durchzuführen. Im Batch-Verfahren wird der gesamte Rohstoff in Behälter gegeben, angeimpft und mit Belüftung fermentiert, ohne dass vor dem Abziehen des Mycels weiterer Rohstoff zugegeben wird. Im Fed-batch-Verfahren wird nur ein Teil des Rohstoffs in Behälter gegeben, angeimpft und mit Belüftung fermentiert, wobei bis zum Abziehen des Mycels optional satzweise oder kontinuierlich weiterer Rohstoff zugegeben wird. Optional werden Ammoniumionen kontinuierlich oder intermittierend während der Fermentation zugesetzt, als Pulver oder Lösung.

Generell und insbesondere bei den nachfolgenden Beispielen beziehen sich Angaben zur Trockenmasse (TS) auf Myzelium, z.B. bestimmt nach Waschen von Myzelium mit Wasser, um Bestandteile des Nährmediums vom Myzelium zu entfernen.

### Beispiel 1: submers fermentative Herstellung von Aspergillus oryzae

Als Substrat wurde der unlösliche Rückstand aus der Herstellung von Tofu aus Sojabohnen, der auch Okara genannt wird, zu 10 Gew.-% in Wasser suspendiert, um ein Nährmedium herzustellen. Die Suspension wurde mit nachfolgenden Mikroorganismen, die natürlicherweise in solchen Nebenströmen vorkommen können, inokuliert, um ein beispielhaftes Substrat mit Kontamination durch Bakterien und Schimmelpilze herzustellen: 10⁵ Sporen/mL von Bacillus cereus, je ca. 10⁵ Sporen/mL von Rhizopus arrhizus und Mucor racemosus, ca. 10⁵ KbE/mL von Lactiplantibacillus plantarum und ca. 10⁵ KbE/mL von Salmonella enterica serovar. Typhimurium.

Bei Absenkung des pH-Werts des Mediums von anfänglich ca. 6 bis 8 durch Zusetzen von Säure, bevorzugt wässrigen Rückstands, der aus Fermentationsbrühe abgetrennt war, auf pH 3,0 mit anschließendem Pasteurisieren bei 80 °C für 120 min wurden Sporen von Bacillus cereus um ca. 3 x 10³4 KbE/mL vermindert, bei der anschließenden aeroben Inkubation mit Durchmischen um eine weitere Zehnerpotenz. Die Sporen von Rhizopus arrhizus und Mucor racemosus sowie L. plantarum und S. enterica waren nach der Fermentation vollständig inaktiviert.

Zur aeroben Fermentation wurden in mit Luft begasten Behältern Aliquots des Nährmediums ohne Einstellung des pH-Werts mit Zusetzen von 10 g/L Ammoniumsulfat (pH nicht eingestellt) oder nach Ansäuern mit A. oryzae inokuliert. Der pH-Wert des Mediums bzw. der Fermenterbrühe wurde zum Zeitpunkt des Inokulierens (pH-Wert 0 h) und nach 48 h Belüften (pH-Wert 48 h) bestimmt, die Trockensubstanz des abgetrennten Pilzmycels (TS) wurde nach 48 h bestimmt. Eine Steuerung des pH-Werts, z.B. durch Zugabe von Säure, wurde nicht eingesetzt.

Die Ergebnisse zeigen, dass das Nährmedium mit zugesetztem Ammoniumsulfat vom anfänglichen pH-Wert um ca. 4 pH-Einheiten allein durch das Wachstum des Pilzes (7,29 g/L TS) gesäuert wurde. Bei Einstellen des anfänglichen pH-Werts auf pH 4 (A1, A2, A3) oder auf pH 3 (B1, B2, B3), ohne Zusatz von Ammoniumsulfat zum Nährmedium wurde nur eine geringe weitere Säuerung durch das Pilzwachstum erreicht, bei ähnlicher oder geringerer Herstellung von Pilz-TS.

| **Ansatz** | **pH-Wert 0 h** | **pH-Wert 48h** | **TS [g/L]** |
|---|---|---|---|
| pH nicht eingestellt | 7,86 | 3,94 | 7,29 |
| A1 pH 4 | 4,44 | 3,68 | 7,41 |
| A2 pH 4 | 4,38 | 3,66 | 6,80 |
| A3 pH 4 | 4,38 | 3,66 | 7,06 |
| B1 pH 3 | 3,24 | 2,93 | 7,45 |
| B2 pH 3 | 3,22 | 2,95 | 6,30 |
| B3 pH 3 | 3,21 | 2,90 | 6,68 |

### Beispiel 2: submerse fermentative Herstellung von A. oryzae (White Koji) mit verschiedenen Substraten

Verschiedene Substrate, darunter der unlösliche Rückstand aus der Herstellung von Tofu aus Sojabohnen (Sojaokara), der unlösliche Rückstand aus der Herstellung von Tofu aus Hafer (Haferokara), der unlösliche Rückstand aus der Herstellung von Tofu aus Reis (Reisokara), Hafermehl, Weizenmehl, Roggenkleie, Dinkelkleie oder Erbsenstärke wurden mit je 10 g/L Ammoniumsulfat oder ohne Ammoniumsulfat zu je 30 g/L in Wasser eingemischt. Dieses Nährmedium wurde nicht pasteurisiert oder einer keimreduzierenden Behandlung unterzogen. Der pH-Wert zu Beginn der Inokulation und nach 48 h wurde in je zwei parallel angesetzten Behältern gemessen. Zur Inokulation wurden 10 bis 20 mL einer Vorkultur, die mit 10³ bis 10⁹ Sporen/mL in LB Medium inokuliert und für 24 h im Schüttelkolben bei 30 bis 37 °C inkubiert wurde, und/oder eine Vorkultur aus Myzelium von A. oryzae, z.B. 30 - 120 mL mit 0,2 bis 0,8 gTS/L, bevorzugt mittels Utra-Turrax zerkleinert, zugesetzt, zur Inokulation von 0,5 bis 1,5 L Nährmedium. Die Fermentation erfolgte in mit Luft begasten Behältern. Eine Steuerung des pH-Werts, z.B. durch Zugabe von Säure, wurde nicht eingesetzt.

Die Ergebnisse zeigen, dass auch bei unterschiedlichen pflanzlichen Substraten allein der Zusatz von Ammoniumsulfat die Wirkung hat, dass der pH-Wert der Fermenterbrühe um 3 bis 5 pH-Einheiten abgesenkt wird.

| **Nährmedium** | **pH-Wert 0 h** | | **pH-Wert 48 h** | |
|---|---|---|---|---|
| | **Behälter 1** | **Behälter 2** | **Behälter 1** | **Behälter 2** |
| 30 g/L Hafermehl + 10 g/L Ammoniumsulfat | 7,22 | 7,09 | 2,47 | 2,50 |
| 30 g/L Weizenmehl + 10 g/L Ammoniumsulfat | 7,54 | 7,54 | 2,34 | 2,42 |
| 30 g/L Sojaokara + 10 g/L Ammoniumsulfat | 7,39 | 7,37 | 3,32 | 4,69 |
| 30 g/L Haferokara + 10 g/L Ammoniumsulfat | 7,14 | 7,10 | 3,39 | 3,76 |
| 30 g/L Reisokara + 10 g/L Ammoniumsulfat | 7,13 | 7,14 | 3,70 | 3,91 |
| 30 g/L Weizenkleie + 10 g/L Ammoniumsulfat | 6,38 | 6,35 | 3,73 | 3,89 |
| 30 g/L Roggenkleie + 10 g/L Ammoniumsulfat | 6,43 | 6,44 | 3,13 | 3,14 |
| 30 g/L Dinkelkleie + 10 g/L Ammoniumsulfat | 6,26 | 6,25 | 3,59 | 3,86 |
| 30 g/L Erbsenstärke + 10 g/L Ammoniumsulfat | 7,37 | 7,31 | 2,61 | 2,54 |
| 30 g/L Hafermehl | 7,89 | 7,95 | 3,84 | 4,02 |
| 30 g/L Weizenmehl | 7,80 | 7,98 | 4,14 | 4,19 |
| 30 g/L Sojaokara | 7,74 | 7,75 | 6,22 | 5,88 |
| 30 g/L Haferokara | 7,07 | 7,13 | 6,47 | 6,44 |
| 30 g/L Reisokara | 6,77 | 6,98 | 6,36 | 6,32 |
| 30 g/L Weizenkleie | 6,48 | 6,52 | 5,99 | 5,98 |
| 30 g/L Roggenkleie | 6,45 | 6,51 | 5,70 | 5,88 |
| 30 g/L Dinkelkleie | n.b. | n.b. | 5,87 | 5,76 |
| 30 g/L Erbsenstärke | 7,98 | 8,05 | 3,80 | 3,88 |

Zum Vergleich wurde in je zwei parallel angesetzten Behältern Nährmedium aus 30 g/L Sojaokara mit 10 g/L Ammoniumsulfat, 10 g/L Harnstoff, 10 g/L Natriumnitrat oder 20 g/L Natriumnitrat als zusätzlicher Stickstoffquelle versetzt und nach Inokulieren mit 30 - 120 mL Myzelium von A. oryzae (*white Koji*) mit einem Gehalt von 0,2 bis 0,8 gTS/L, mittels Ultra-Turrax zerkleinert, für 48 h aerob fermentiert. Die Ergebnisse zeigen, dass von diesen zugesetzten Stickstoffquellen nur Ammoniumionen, insbesondere Ammoniumsulfat die Absenkung des pH-Werts der Fermentationsbrühe während der aeroben Fermentation hat, während der pH-Wert bei Nitrat oder Harnstoff nur unwesentlich durch die Fermentation beeinflusst wurde.

| **Nährmedium** | **pH-Wert 0 h** | | **pH-Wert 48 h** | |
|---|---|---|---|---|
| | **Behälter 1** | **Behälter 2** | **Behälter 1** | **Behälter 2** |
| 30 g/L Sojaokara + 10 g/L Ammoniumsulfat | 7,39 | 7,37 | 3,32 | 4,69 |
| 30 g/L Sojaokara + 10 g/L Harnstoff | 9,26 | 9,25 | 9,02 | 9 |
| 30 g/L Sojaokara + 10 g/L Natriumnitrat | 8,16 | 7,48 | 7,24 | 6,41 |
| 30 g/L Sojaokara + 20 g/L Natriumnitrat | 7,61 | 7,58 | 7,1 | 7,16 |

Die Wirkung von Ammoniumionen, dass durch die Fermentation der pH-Wert deutlich abgesenkt wird, zeigt sich auch bei längerer Dauer der Fermentation. Die nachfolgenden Zusammensetzungen von Nährmedium wurden, jeweils in 2 Behältern, ohne Pasteurisation und ohne Einstellung des pH-Werts mit Aspergillus oryzae inokuliert. Der pH-Wert wurde zu Beginn der Inokulation (0 h), nach 2 Tagen (48 h) und nach 5 Tagen (120 h) gemessen.
Behälter 1 + 2: Hafermehl 20g/L, Saccharose 10 g/L, Ammoniumsulfat 10 g/L
Behälter 3 + 4: Hafermehl 20g/L, Saccharose 10 g/L
Behälter 5 + 6: Hafermehl 20g/L, Saccharose 10 g/L, Frischhefe (Saccharomyces cerevisiae) 10 g/L

| Behälter | pH-Wert 0 h | pH-Wert 48 h | pH-Wert 120 h |
|---|---|---|---|
| 1 | 5,5 | 2,61 | 2,06 |
| 2 | 5,5 | 2,75 | 2,05 |
| 3 | 5,8 | 3,95 | 4,77 |
| 4 | 5,8 | 3,75 | 4,79 |
| 5 | 5,8 | 5,51 | 5,98 |
| 6 | 5,8 | 5,53 | 6,03 |

Auch diese Ergebnisse zeigen, dass bei Zusatz von Ammoniumionen zum Nährmedium der pH-Wert allein durch die aerobe Fermentation um zumindest 2 pH-Einheiten erniedrigt wird.

## Patentansprüche

1. Verfahren zur fermentativen Herstellung von Pilzmycel mit den Schritten
1) Bereitstellen eines wässrigen Nährmediums mit einem Gehalt an Substrat, das pflanzliches Protein enthält,
2) Inokulieren des Nährmediums mit zumindest einem Pilz zur Erzeugung eines mit dem zumindest einen Pilz inokulierten Nährmediums,
3) Durchmischen und Belüften des inokulierten Nährmediums zum Anziehen von Pilzmasse des zumindest einen Pilzes zur fermentativen Herstellung von Fermenterbrühe, die angezogene Pilzmasse enthält, wobei dem Nährmedium Ammoniumionen zu 0,02 bis 0,1 M zugesetzt werden, wobei der pH-Wert durch die Fermentation um zumindest 2 pH-Einheiten erniedrigt wird,
4) ohne kontrolliertes Einstellen des pH-Werts,
5) Abtrennen von Pilzmasse aus der Fermenterbrühe zur Herstellung wässriger Pilzmasse und zumindest eines wässrigen Rückstands,
6) Bereitstellen eines weiteren wässrigen Nährmediums durch Mischen zumindest eines Teils des wässrigen Rückstands, der optional unlösliche pflanzliche Bestandteile enthält oder aus löslichen Bestandteilen besteht, mit pflanzlichem Protein,
und
Wiederholung der Schritte 2) bis 6)
aufweist.

2. Verfahren nach Anspruch 1, **dadurch gekennzeichnet, dass** in Schritt 3) während des Anziehen der Pilzmasse im Nährmedium mit zugesetzten Ammoniumionen der pH-Wert um zumindest 4 pH-Einheiten erniedrigt wird.

3. Verfahren nach einem der voranstehenden Ansprüche, **dadurch gekennzeichnet, dass** Ammoniumionen als Ammoniumsulfat zugesetzt werden.

4. Verfahren nach einem der voranstehenden Ansprüche, **dadurch gekennzeichnet, dass** das Substrat der wasserunlösliche Rückstand aus der wässrigen Extraktion von Bohnen, insbesondere Sojabohnen, Hafer, Weizen, Reis und/oder Linsen ist.

5. Verfahren nach einem der voranstehenden Ansprüche, **dadurch gekennzeichnet, dass** der pH-Wert der Fermenterbrühe durch das Anziehen des Pilzes innerhalb von 48 h auf einen pH-Wert von 2,5 bis 4,5 erniedrigt wird.

6. Verfahren nach einem der voranstehenden Ansprüche, **dadurch gekennzeichnet, dass** das Nährmedium, das Ammoniumionen, Substrat und wässrigen Rückstand enthält, vor dem Inokulieren pasteurisiert wird.

7. Verfahren nach einem der voranstehenden Ansprüche, **dadurch gekennzeichnet, dass** das Substrat und/oder das Nährmedium einen Gehalt an Bakterien von maximal 10⁶ KbE/mL aufweist.

8. Verfahren nach einem der voranstehenden Ansprüche, **dadurch gekennzeichnet, dass** vor Schritt 2) der Gehalt an Bakterien des Substrats und/oder des Nährmediums durch Pasteurisation oder Teilentkeimung auf maximal 10⁶ KbE/mL verringert wird.

9. Verfahren nach einem der voranstehenden Ansprüche, **dadurch gekennzeichnet, dass** das Pasteurisieren durch Erwärmen auf 68 °C bis 120 °C für 20 bis 120 min, bevorzugt bis 60 min erfolgt.

10. Verfahren nach einem der voranstehenden Ansprüche, **dadurch gekennzeichnet, dass** der Pilz Aspergillus oryzae ist.
